(19) **Europäisches Patentamt**

**European Patent Office**

**Office européen des brevets**

(11) **EP 1 574 478 A1**

(12) **EUROPÄISCHE PATENTANMELDUNG**

| | |
|---|---|
| (43) Veröffentlichungstag:<br>**14.09.2005  Patentblatt 2005/37** | (51) Int Cl.7: **C01B 31/28** |

(21) Anmeldenummer: **04005421.5**

(22) Anmeldetag: **08.03.2004**

| | |
|---|---|
| (84) Benannte Vertragsstaaten:<br>**AT BE BG CH CY CZ DE DK EE ES FI FR GB GR HU IE IT LI LU MC NL PL PT RO SE SI SK TR**<br>Benannte Erstreckungsstaaten:<br>**AL LT LV MK** | • **Eicher, Johannes, Dr.**<br>**31319 Sehnde-Ilten (DE)** |
| (71) Anmelder: **Solvay Fluor GmbH**<br>**30173 Hannover (DE)** | (74) Vertreter: **Fischer, Reiner**<br>**Solvay Fluor und**<br>**Derivate GmbH & Co. KG**<br>**Hans-Böckler-Allee 20**<br>**30173 Hannover (DE)** |
| (72) Erfinder:<br>• **Braun, Max, Dr.**<br>**30900 Wedemark (DE)** | |

(54) **Herstellung von Carbonylfluorid**

(57)    Carbonylfluorid, das auch als Ätzgas verwendet werden kann, lässt sich durch photochemische Oxidation von Chlordifluormethan mit Licht, beispielsweise mit Licht einer Wellenlänge ≥ 280 nm in Anwesenheit von Chlor herstellen.

EP 1 574 478 A1

**Beschreibung**

Die Erfindung bezieht sich auf die Herstellung von Carbonylfluorid (Fluorphosgen) durch photochemische Oxidation.

Carbonylfluorid ist als neues Ätzgas für die Reinigung von CVD-Reaktoren vorgeschlagen worden. Die technische Herstellung ist durch Erhitzen eines Monohalodifluormethans möglich, siehe EP-A-0 310255. In wissenschaftlichen Publikationen ist auch die photochemische Oxidation von Chlordifluormethan in Anwesenheit von Chlor beschrieben worden, siehe E. O. Edney und D. J. Driscoll, Int. Journal of Chemical Kinetics, Vol. 24(1992), Seiten 1067 bis 1081.Der Druck betrug 700 Torr. Ziel war es, Erkenntnisse über die troposphärische Zersetzung verschiedener Halogenkohlenwasserstoffe zu erhalten.

Aufgabe der vorliegenden Erfindung war es, ein technisch vorteilhaft durchführbares Verfahren zur Herstellung von Carbonylfluorid, $C(O)F_2$, anzugeben. Diese Aufgabe wird durch das Verfahren der vorliegenden Erfindung gelöst.

Das erfindungsgemäße Verfahren sieht die Herstellung von $C(O)F_2$ durch Photooxidation von $CHClF_2$ mit Sauerstoff vor. Dabei wird Licht eingestrahlt, das nicht aus einer einzigen Wellenlänge besteht, sondern einen Spektralbereich aufweist, der mindestens 50 nm umfasst (d.h. der Lichtanteil mit niedrigster Wellenlänge und der Lichtanteil mit der höchsten Wellenlänge liegen mindestens 50 nm auseinander).

Der Druck im Reaktor entspricht bevorzugt mindestens dem Umgebungsdruck, also 1 bar (abs.). Er kann auch darüber liegen. Der Druck liegt bevorzugt im Bereich von 1 bar (abs.) bis 11 bar (abs.). Die Temperatur liegt bevorzugt im Bereich von 20 bis 300 °C, besonders im Bereich von 30 bis 300 °C und insbesondere im Bereich von 30 bis 90 °C. Vorteilhaft wählt man die Bedingungen hinsichtlich Druck und Temperatur derart, dass das Reaktionsgemisch gasförmig bleibt.

Ganz besonders bevorzugt arbeitet man drucklos. Der Begriff "drucklos" bedeutet im Rahmen der vorliegenden Erfindung, dass auf die Reaktionsmischung außer dem Umgebungsdruck (d.h. etwa 1 bar), dem Förderdruck des Sauerstoffgases (bzw. des sauerstoffhaltigen Gases, man kann z.B. Luft oder Sauerstoff/Inertgas-Gemische einsetzen) und des gegebenenfalls eingesetzten Chlors sowie dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als der Umgebungsdruck.

Das Verfahren kann batchweise oder bevorzugt kontinuierlich durchgeführt werden. Vorzugsweise geht man so vor, dass man kontinuierlich Ausgangsmaterial (das entsprechende Edukt, Sauerstoff bzw. ein Sauerstoff enthaltendes Gas wie Luft oder reinen Sauerstoff und gegebenenfalls Chlor) in eine Durchflussapparatur einspeist und entsprechend der eingespeisten Menge kontinuierlich Reaktionsprodukt abzieht. Die durchschnittliche Verweilzeit im Reaktionsgefäß liegt vorteilhaft zwischen 0,01 und 30 Minuten, bevorzugt zwischen 0,1 bis 3 min, besonders bevorzugt zwischen 0,3 und 1,5 Minuten. Die optimale durchschnittliche Verweilzeit, die u.a. von der Art der Lampen, der Strahlungsleistung der Lampen und von geometrischen Parametem der Bestrahlungsapparatur abhängig ist, kann man durch einfache Handversuche und Analyse des Produktstroms, beispielsweise durch Gaschromatographie, ermitteln. Vorteilhaft kann es auch sein, die Reaktionsmischung beispielsweise durch geeignete Einbauten im Reaktor gut zu verwirbeln. Die optimale Verweilzeit bei batchweiser Durchführung kann in gleicher Weise ermittelt werden.

Das Verfahren kann in zwei bevorzugten Ausführungsformen durchgeführt werden.

Eine Ausführungsform sieht die Photooxidation in Abwesenheit von Chlor oder anderen Radikalinitiatoren oder Aktivatoren vor. Beispielsweise kann die Bestrahlung durch Quarzglas hindurch vorgenommen werden; andere Bauteile des Reaktors, die nicht zwischen Lichtquelle und Reaktionsgemisch angeordnet sind, können natürlich aus beliebigen Bauteilen, z.B. auch aus Borsilikat-Glas sein, welches bestimmte Strahlungsanteile filtert (siehe unten). Als Strahler eigenen sich übliche Strahler, die beispielsweise Strahlung im Bereich von 250 bis 400 nm oder sogar bis 600 nm abgeben (das Spektrum kann auch über die untere oder obere Grenze hinausgehen).

Eine weitere, besonders bevorzugte Ausführungsform sieht die Bestrahlung in Anwesenheit von elementarem Chlor unter Bestrahlung mit Licht einer Wellenlänge von $\geq 280$ nm vor, wobei pro Gewichtsteil $CHClF_2$ maximal 0,6 Gewichtsteile elementares Chlor im Reaktionsgemisch enthalten sind. Bevorzugt werden pro Mol $CHClF_2$ 1 bis 50 mol-% Chlor, vorzugsweise 5 bis 20 mol-% elementares Chlor eingesetzt.

Umsatzrate, Ausbeute und Selektivität sind besonders hoch, wenn man in Anwesenheit von elementarem Chlor umsetzt und eine aktivierende Bestrahlung mit Licht einer Wellenlänge $\lambda \geq 280$ nm vornimmt. Frequenzen einer Wellenlänge unterhalb von 280 nm sind dann im Wesentlichen aus dem Frequenzspektrum ausgeblendet. Dies kann man dadurch bewirken, dass man Bestrahlungslampen verwendet, die nur Licht einer Wellenlänge oberhalb oder bei 280 nm abstrahlen, und/oder man verwendet Mittel, die die entsprechenden Frequenzen aus dem abgestrahlten Licht ausblenden. Beispielsweise kann man durch Glas bestrahlen, welches nur für Licht einer Wellenlänge von 280 nm oder darüber durchlässig ist, also den kürzerwelligeren Strahlungsanteil herausfiltert. Gut geeignet dafür sind beispielsweise Borosilikat-Gläser. Geeignete Gläser enthalten beispielsweise 7 bis 13 % $B_2O_3$, 70 bis 80 % $SiO_2$, ferner 2 bis

7 % $Al_2O_3$ und 4 bis 8 % $Na_2O$ + $K_2O$ sowie 0 bis 5% Erdalkalimetalloxide (jeweils Gew.-%). Bekannte Marken für Borosilikat-Gläser sind Duran, Pyrex und Solidex.

Zur Bestrahlung sind besonders gut Bestrahlungslampen geeignet, die nur (UV)Licht einer Wellenlänge oberhalb von oder bei 280 nm abstrahlen. Insbesondere Leuchtstoff-Röhren (z.B. von der Firma Philips) sind sehr gut geeignet. Mit derartigen Lampen kann man die Bestrahlung durch Quarzglas, aber auch durch die vorstehend beschriebenen, den kürzerwelligen Bestrahlungsanteil herausfilternde Gläser vornehmen. Voraussetzung ist natürlich, dass die verwendeten Lampen oder Röhren auch im Absorptionsbereich des elementaren Chlors emittieren. Neben den besonders gut geeigneten Leuchtstoffröhren kann man auch beispielsweise Bestrahlungslampen (z.B. Quecksilber-Mittel- oder Hochdruckstrahler) verwenden; etwaige Linien im Bereich unterhalb von 280 nm werden herausgefiltert, beispielsweise indem man durch ein Glas bestrahlt, das nur für Licht einer Wellenlänge bei und oberhalb von 280 nm durchlässig ist. Verwendbare Gläser sind weiter oben beschrieben. Gut geeignet zur Bestrahlung sind auch Lampen, z.B. Quecksilber-Hochdrucklampen, die aufgrund eines Dotierungsmittels überwiegend oder nur im bevorzugten Wellenbereich bei und oberhalb von 280 nm abstrahlen. Quecksilber-Hochdruckstrahler beispielsweise weisen eine recht intensive Bande im Bereich von 254 nm auf, die, wie oben beschrieben wird, beispielsweise durch Borosilikat-Glas herausgefiltert werden kann. Bei durch Metalljodide dotierten Quecksilber-Hochdruckstrahlern ist diese Linie stark unterdrückt. Überraschend ist die oft überproportionale Erhöhung der Umsatzrate bei Verwendung solcher dotierten Strahler. Besonders gut geeignet sind Quecksilber-Hochdruckstrahler, die mit Galliumjodid, insbesondere Thalliumjodid oder Cadmiumjodid dotiert sind. Auch bei Verwendung solcher dotierter Strahlungslampen filtert man vorteilhaft den kürzerwelligen Strahlungsanteil mit $\lambda < 280$ nm heraus, beispielsweise indem man in Borosilikat-Glas arbeitet.

Das Molverhältnis zwischen dem Edukt und Sauerstoff kann in einem weiten Bereich schwanken, zweckmäßig setzt man jedoch mindestens 0,4 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,4 bis 1:1, insbesondere 1:0,4 bis 1:0,6 liegt. Der Sauerstoff kann wie gesagt in Form von Luft eingesetzt werden. Bevorzugt setzt man den Sauerstoff in Form eines O2/Inertgas-Gemisches, insbesondere aber als reinen Sauerstoff ein. In Bezug auf die Produktreinheit ist es wünschenswert, dass möglichst wenig Wasser bei der Umsetzung vorhanden ist (beispielsweise weniger als 30 ppm). Gewünschtenfalls kann man die Reaktanten in bekannter Weise von mitgeschlepptem Wasser befreien, z.B. mittels Molekularsieb.

Der Vorteil des erfindungsgemäßen Verfahrens ist die hohe Selektivität und Ausbeute.

Die folgenden Beispiele erläutern die Erfindung, ohne sie einzuschränken.

Beispiel 1: Herstellung von Fluorphosgen ($COF_2$) durch photochemische Reaktion

Reaktionsgleichung:

$$CF_2HCl + ½ O_2 \rightarrow COF_2 + HCl$$

Ansatzgröße:     siehe jeweiliger Versuch

Versuchsdurchführung und Aufbau:

Als Reaktionsraum diente ein aus Duran-Glas gefertigter Reaktor mit einem Füllvolumen von maximal 580 ml, welcher einen Kühlfinger (Duran) und einen Lampenschacht (Quarzglas) aufwies. Die Gaseinleitung erfolgte über eine Glasfritte, die sich am Reaktorboden befand. Der Quecksilberdampf-Hochdruckstrahler wurde mit Pressluft gekühlt.

Zu Versuchsbeginn wurde zuerst die Pressluftkühlung aufgedreht und dann die Lampe gezündet. Nach ca. 10 min hat der Strahler seine Leistung (500 oder 700Watt) erreicht. Es wurde jetzt die Einleitung der Gase begonnen. Zunächst wurde die Einleitung von HCFC-22 (R 22) gestartet, dann die Einleitung des Chlors, schließlich auch noch die Einleitung des Sauerstoffs, so dass alle drei Reaktanten in den Reaktor eingespeist wurden.

Alle Gase wurden dann in einem bestimmten Verhältnis zugleich dosiert und durch den Reaktorraum geleitet. Der entstehende Produktgasstrom wurde durch eine Waschflasche (gefüllt mit ca. 5%iger $H_2O_2$- Lösung) geleitet, um das überschüssige Chlor aufzufangen und in HCl umzuwandeln. Die Proben des Produktgasstroms wurden vor der Waschflasche entnommen.

Versuch 1:

| Ansatz | 0,5 mol R22/h |
|---|---|
|  | 0,5 mol $O_2$/h |
|  | wenig $Cl_2$ |
| Durchführung | Lampenleistung bei 700 Watt |

| Probenahme und Uhrzeit (Beginn 7:10) | R22 (in g) | R22 mol/h | Cl2 (in g) | Cl2 mol/h | O2 (in g) | O2 mol/h | Verweilzeit im Reaktor (in min) |
|---|---|---|---|---|---|---|---|
| 07:30 | 17,2 | 0,6 | 2 | 0,08 | 4,8 | 0,5 | 1,23 |
| 07:45 | 37,7 | 0,9 | 2,7 | 0,04 | 9,6 | 0,6 | 0,94 |
| 08:10 | 54,6 | 0,5 | 4,8 | 0,07 | 14,6 | 0,4 | 1,49 |

Analysenauswertung der Gasproben (alle Analysen ohne Luft berechnet):

| Probennahme: | | | |
|---|---|---|---|
| um 7.45 Uhr | 45,1% $COF_2$ | um 8.10 Uhr | 24,5% $COF_2$ |
|  | 44,2% HCl |  | 23,7% HCl |
|  | 8,6% $CO_2$ |  | 10,9% $CO_2$ |
|  | 1,8% R12 |  | 3,9% R12 |
|  | 0,3% $H_2O$ |  | 37,0% R22 |

Versuch 2:

| Ansatz | 0,5 mol R22/h |
|---|---|
|  | 0,5 mol $O_2$/h |
|  | wenig $Cl_2$ |
| Durchführung | Lampenleistung bei 500 Watt |

| Probenahme und Uhrzeit Beginn 7:30 | R22 (in g) | R22 mol/h | Cl2 (in g) | Cl2 mol/h | O2 (in g) | O2 mol/h | Verweilzeit im Reaktor (in min) |
|---|---|---|---|---|---|---|---|
| 07:50 | 20,7 | 0,7 | 1,8 | 0,08 | 5,8 | 0,5 | 1,13 |
| 08:45 | 80 | 0,6 | 3,8 | 0,03 | 21,7 | 0,5 | 1,28 |
| 09:45 | 130,8 | 0,6 | 11,3 | 0,1 | 38,9 | 0,5 | 1,21 |
| 11:15 | 220,3 | 0,7 | 14,2 | 0,03 | 56,9 | 0,4 | 1,28 |
| 12:00 | 264,5 | 0,7 | 18,3 | 0,08 | 75,8 | 0,8 | 0,92 |
| 13:00 | 303,7 | 0,5 | 22,0 | 0,1 | 84,4 | 0,3 | 1,71 |
| 13:30 | 342,9 | 0,9 | 0 | 0 | 97,9 | 0,8 | 0,85 |

Analysenauswertung: (alle Analysen ohne Luft berechnet, ausgenommen die Probe um 13.30 Uhr):

| Probennahme: | | | |
|---|---|---|---|
| um 7.50 Uhr | 32,9% $COF_2$<br>34,3% HCl<br>5,5% $CO_2$<br>8,6% R12<br>0,3% $H_2O$<br>18,4% R22 | 8.45 Uhr | 43,1% $COF_2$<br>42,7% HCl<br>6,1 % $CO_2$<br>6,5% R12<br>0,8% R22 |
| um 9.45 Uhr | 44,6% $COF_2$<br>41,6% HCl<br>3,1% $CO_2$<br>6,8% R12<br>4,0% R22 | 11.15 Uhr | 45,6% $COF_2$<br>43,9% HCl<br>5,7% $CO_2$<br>3,6% R12<br>1,3% R22 |
| um 12.00 Uhr | 44,9% $COF_2$<br>40,3% HCl<br>11,8% $CO_2$<br>2,6% R12<br>0,3% R22 | um 13.00 Uhr | 42,0% $COF_2$<br>41,8% HCl<br>13,9% $CO_2$<br>1,7% R12<br>0,5% $H_2O$ |
| um 13.30 Uhr | 49,3% Luft ($O_2$)<br>44,0% R22<br><br>2,2% HCl<br>2,2% $CO_2$<br>2,0% $COF_2$<br>0,2% $H_2O$ | | |

Versuch 3:

| Ansatz | 0,5 mol R22/h |
|---|---|
| | 0,5 mol $O_2$/h |
| | wenig $Cl_2$ |
| Durchführung | Lampenleistung bei 500 Watt |

| Probenahme<br>und Uhrzeit<br>(Beginn 7:45) | R22 (in g) | R22 mol/h | Cl2 (in g) | Cl2 mol/h | O2 (in g) | O2 mol/h | Verweilzeit im<br>Reaktor (in min) |
|---|---|---|---|---|---|---|---|
| 08:45 | 61,9 | 0,7 | 4,7 | 0,07 | 16,3 | 0,5 | 1,14 |
| 09:45 | 118,8 | 0,7 | 8,6 | 0,06 | 33 | 0,5 | 1,15 |
| 11:15 | 205,7 | 0,7 | 12,5 | 0,04 | 58,8 | 0,5 | 1,17 |
| 11:45 | 242,6 | 0,9 | 12,7 | 0,006 | 65,6 | 0,4 | 1,11 |

Analysenauswertung (alle Analysen ohne Luft berechnet):

| Probennahme: | | | |
|---|---|---|---|
| um 8.45 Uhr: | 43,6% $COF_2$ <br> 42,3% HCl <br> 10,7% $CO_2$ <br> 1,7% R12 <br> 1,0% R22 <br> 0,6% $H_2O$ | um 9.45 Uhr: | 46,0% $COF_2$ <br> 43,2% HCl <br> 6,9% $CO_2$ <br> 1,2% R12 <br> 2,2% R22 <br> 0,6% $H_2O$ |
| um 11.15 Uhr | 36,7% $COF_2$ <br> 38,4% HCl <br> 8,4% $CO_2$ <br> 0,9% R12 <br> 15,4% R22 <br> 0,2% $H_2O$ | um 11.45 Uhr | 41,7% $COF_2$ <br> 40,7% HCl <br> 7,2% $CO_2$ <br> 0,9% R12 <br> 9,3% R22 <br> 0,3% $H_2O$ |

Beispiel 2: Herstellung von Fluorphosgen ($COF_2$) durch photochemische Reaktion (mit Quarzglaskühlfinger und ohne $Cl_2$)

Versuchsdurchführung und Aufbau:

Als Reaktionsraum dient ein aus Duran-Glas gefertigter Reaktor mit einem Füllvolumen von maximal 580ml, welcher einen aus Quarz gefertigten Kühlfinger und einen Lampenschacht (Quarzglas) aufwies. Die Gaseinleitung erfolgte über eine Glasfritte, die sich am Reaktorboden befand. Der Quecksilberdampf- Hochdruckstrahler wurde mit Pressluft gekühlt. Zu Versuchsbeginn wurde zuerst die Pressluftkühlung aufgedreht und dann die Lampe gezündet. Nach ca. 10 min hatte der Strahler seine Leistung erreicht. Zunächst wurde HCFC-22 in den Reaktor eingeleitet und dann der Sauerstoff zugeschaltet.
Die beiden Gase wurden nun in einem bestimmten Verhältnis zugleich dosiert und durch den Reaktorraum geleitet. Der entstehende Produktgasstrom wurde analysiert.

Versuch 2.1:

| Ansatz | 0,5 mol R22/h <br> 0,4 mol $O_2$/h |
|---|---|
| Durchführung | Lampenleistung bei 500 Watt |

| Probenahme und Uhrzeit (Beginn 9:00) | R22 (in g) | R22 mol/h | O2 (in g) | O2 mol/h | Verweilzeit im Reaktor (in min) |
|---|---|---|---|---|---|
| 09:30 | 29,5 | 0,68 | 11,5 | 0,70 | 1,05 |
| 10:00 | 43,8 | 0,51 | 19,0 | 0,59 | 1,32 |
| 10:30 | 62,5 | 0,43 | 26,0 | 0,44 | 1,67 |
| 11:00 | 83,6 | 0,49 | 35,0 | 0,56 | 1,38 |
| 11:30 | 102,3 | 0,43 | 40,0 | 0,31 | 1,96 |
| 12:00 | 120,2 | 0,41 | 45,5 | 0,34 | 1,93 |

(fortgesetzt)

| Probenahme und Uhrzeit (Beginn 9:00) | R22 (in g) | R22 mol/h | O2 (in g) | O2 mol/h | Verweilzeit im Reaktor (in min) |
|---|---|---|---|---|---|
| 13:00 | 157,1 | 0,43 | 55,5 | 0,31 | 1,96 |
| 13:30 | 176,3 | 0,44 | 61,0 | 0,34 | 1,86 |

Analysenauswertung:

| Probennahme: | | | |
|---|---|---|---|
| um 9.30 Uhr | 56,2% $O_2$<br>15,6% $COF_2$<br>9,7% HCl<br>1,3% $CO_2$<br>16,6% R22<br>0,4% $H_2O$<br>0,24% COFCl | 10.00 Uhr | 38,8% $COF_2$<br>34,7% HCl<br>7,7% $CO_2$<br>14,2% R22<br>0,4% $H_2O$<br>3,7% COFCl<br>0,6% R12<br>0,04% $COCl_2$ |
| Um 10.30 Uhr | 35,9% $COF_2$<br>31,3% HCl<br>6,1% $CO_2$<br>21,4% R22<br>0,2% $H_2O$<br>4,5% COFCl<br>0,6% R12<br>0,05% $COCl_2$ | um 11.00 Uhr | 35,4% $COF_2$<br>32,3% HCl<br>7,1% $CO_2$<br>18,6% R22<br>5,7% COFCl<br>0,8% R12<br>0,07% $COCl_2$ |
| um 11.30 Uhr | 33,6% $COF_2$<br>33,7% HCl<br>8,1% $CO_2$<br>18,6% R22<br>5,7% COFCl<br>0,7% R12<br>0,1% $COCl_2$ | um 12.00 Uhr | 31,2% $COF_2$<br>29,9% HCl<br>7,9% $CO_2$<br>24,4% R22<br>5,7% COFCl<br>0,9% R12<br>0,1% $COCl_2$ |
| um 13.00 Uhr | 30,9% $COF_2$<br>28,0% HCl<br>6,8% $CO_2$<br>27,3% R22 | um 13.30 Uhr | 27,1% $COF_2$<br>30,4% HCl<br>11,5% $CO_2$<br>23,5% R22 |

(fortgesetzt)

| Probennahme: | | | |
|---|---|---|---|
| | 0,2% $H_2O$<br>5,9% COFCl<br>0,7% R12<br>0,1% $COCl_2$ | | 6,4% COFCl<br>1,0% R12<br>0,2% $COCl_2$ |

Die Beispiele belegen, dass besonders gute Ausbeute und Umsatz bei der Durchführung in Anwesenheit von Chlor und mit Licht erreicht werden, dessen kürzerwelliger Anteil (λ<280 nm) herausgefiltert ist.
die Isolierung des Carbonylfluorids kann nach üblichen Methoden erfolgen, beispielsweise durch Tieftemperatur- oder Druckdestillation.

**Patentansprüche**

1.   Verfahren zur Herstellung von $C(O)F_2$ durch Photooxidation von $CHClF_2$ mit Sauerstoff.

2.   Verfahren nach Anspruch 1, **dadurch gekennzeichnet, dass** man die Bestrahlung in Abwesenheit von Chlor vornimmt und Licht einstrahlt, das auch Wellenlängen < 280 nm aufweist, oder dass man die Bestrahlung in Anwesenheit von elementarem Chlor mit Licht einer Wellenlänge von ≥ 280 nm, wobei pro Mol $CHClF_2$ maximal 50 Mol.-% elementares Chlor im Reaktionsgemisch enthalten sind.

3.   Verfahren nach Anspruch 1, **dadurch gekennzeichnet, dass** pro Mol $CHClF_2$ 5 bis 20 mol.-% elementares Chlor enthalten sind.

4.   Verfahren nach Anspruch 1, **dadurch gekennzeichnet, dass** man die Bestrahlung bei einer Temperatur von 20 bis 300°C, vorzugsweise 30 bis 300 °C, insbesondere 50 bis 90 °C durchführt.

5.   Verfahren nach Anspruch 1, **dadurch gekennzeichnet, dass** man die Bestrahlung bei einem Druck von 1 bis 11 bar (abs.) durchführt.

6.   Verfahren nach Anspruch 1, **dadurch gekennzeichnet, dass** die Reaktanden gasförmig vorliegen.

7.   Verfahren nach Anspruch 1, **dadurch gekennzeichnet, dass** die Umsetzung kontinuierlich durchgeführt wird.

8.   Verfahren nach Anspruch 7, **dadurch gekennzeichnet, dass** die durchschnittliche Verweilzeit im Reaktor zwischen 0,1 und 3 Minuten liegt.

**Europäisches Patentamt**

## EUROPÄISCHER RECHERCHENBERICHT

Nummer der Anmeldung

EP 04 00 5421

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DATABASE CHEMABS [Online] CHEMICAL ABSTRACTS SERVICE, COLUMBUS, OHIO, US; KUZ'MENKO, V. A.: "Reactions of carbon difluoride and oxygen. Chlorinecatalysis" XP002296381 gefunden im STN Database accession no. 112:14054 * Zusammenfassung * & ZHURNAL FIZICHESKOI KHIMII , 63(7), 1911-12 CODEN: ZFKHA9; ISSN: 0044-4537, 1989, ----- | 1-8 | C01B31/28 |
| X | ATKINSON R ET AL: "TROPHOSPHERIC AND STRATOSPHERIC SINKS FOR HALOCARBONS: PHOTOOXIDATION, O(1D) ATOM, AND OH RADICAL REACTIONS" JOURNAL OF GEOGRAPHICAL RESEARCH, RICHMOND, VA, US, Bd. 81, Nr. 33, 20. November 1976 (1976-11-20), Seiten 5765-5770, XP008032999 ISSN: 0148-0227 * Zusammenfassung * ----- -/-- | 1 | |

| RECHERCHIERTE SACHGEBIETE (Int.Cl.7) |
|---|
| C01B |

Der vorliegende Recherchenbericht wurde für alle Patentansprüche erstellt

| Recherchenort | Abschlußdatum der Recherche | Prüfer |
|---|---|---|
| Den Haag | 15. September 2004 | Rigondaud, B |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

...........................................................................................
& : Mitglied der gleichen Patentfamilie, übereinstimmendes
Dokument

EPO FORM 1503 03.82 (P04C03)

**Europäisches Patentamt**

# EUROPÄISCHER RECHERCHENBERICHT

Nummer der Anmeldung

EP 04 00 5421

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,X | EDNEY E O ET AL: "CHLORINE INITIATED PHOTOOXIDATION STUDIES OF HYDROCHLOROFLUOROCARBONS (HCFCS) AND HYDROFLUOROCARBONS (HFCS): RESULTS FOR HCFC-22 (CHCLF2); HFC-41 (CH3F); HCFC-124 NCCIFHCF3); HFC-125 (CF3CHF2); HFC-134A (CF3CH2F); HCFC-142B (CCLF2CH3); AND HFC-152A (CHF2" INTERNATIONAL JOURNAL OF CHEMICAL KINETICS, WILEY, NEW YORK, NY, US, Bd. 24, Nr. 12, 1992, Seiten 1067-1081, XP008032981 ISSN: 0538-8066 * Zusammenfassung * * Seite 1068 - Seite 1069 * * Seite 1071 * ----- | 1 | |
| X | BROWNSWORD RICHARD A ET AL: "Photodissociation dynamics of CHF2Cl after photoexcitation at the Lyman-alpha wavelength (121.6 nm)" J PHYS CHEM A; JOURNAL OF PHYSICAL CHEMISTRY A MOLECULES FEB 6 1997 ACS, WASHINGTON, DC, USA, Bd. 101, Nr. 6, 6. Februar 1997 (1997-02-06), Seiten 995-999, XP002296380 * das ganze Dokument * ----- | 1 | RECHERCHIERTE SACHGEBIETE (Int.Cl.7) |
| A | DATABASE CHEMABS [Online] CHEMICAL ABSTRACTS SERVICE, COLUMBUS, OHIO, US; ZHONG, JINXIAN ET AL: "The photolysis characteristics of HCFC-22 in presence of hydrogen peroxide" XP002296382 gefunden im STN Database accession no. 126:218375 * Zusammenfassung * -/-- | 1 | |

Der vorliegende Recherchenbericht wurde für alle Patentansprüche erstellt

| Recherchenort | Abschlußdatum der Recherche | Prüfer |
|---|---|---|
| Den Haag | 15. September 2004 | Rigondaud, B |

EPO FORM 1503 03.82 (P04C03)

EP 1 574 478 A1

| Europäisches Patentamt | EUROPÄISCHER RECHERCHENBERICHT | Nummer der Anmeldung EP 04 00 5421 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| | & HUANJING KEXUE , 17(3), 54-56 CODEN: HCKHDV; ISSN: 0250-3301, 1996, ----- | | |
| A | DATABASE CHEMABS [Online] CHEMICAL ABSTRACTS SERVICE, COLUMBUS, OHIO, US; KOMAROV, V. S. ET AL: "Reaction of ozone with halogen-substituted saturated hydrocarbons" XP002296383 gefunden im STN Database accession no. 93:132037 * Zusammenfassung * & KINETIKA I KATALIZ , 21(2), 519-20 CODEN: KNKTA4; ISSN: 0453-8811, 1980, ----- | 1 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| Den Haag | 15. September 2004 | Rigondaud, B |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

11